# FASCICULE DE BREVET EUROPEEN

(11) **EP 1 106 165 B1**
(45) Date de publication et mention de la délivrance du brevet: **06.05.2004**
(21) Numéro de dépôt: 00403137.3
(22) Date de dépôt: 10.11.2000
(51) Int. Cl.: A61K 7/00, A61K 7/02, A61K 7/48, A61K 7/50

(54) **Composition cosmétique solide chauffante et son utilisation pour le démaquillage de la peau**
Feste wärmende kosmetische Zubereitung und deren Verwendung zum Abschminken
Solid warming cosmetic composition and use thereof for make-up removal

(30) Priorité: 30.11.1999 FR 9915096
(43) Date de publication de la demande: 13.06.2001
(73) Titulaire: L'OREAL, 75008 Paris (FR)
(72) Inventeur: Koely-Therouin, Sandrine, 92140 Clamart (FR); Mattei, Jean-Louis, 92190 Meudon (FR)
(74) Mandataire: Rasson, Catherine

(56) Documents cités:
- EP-A- 0 755 670
- US-A- 5 660 839

## Description

La présente invention a pour objet une composition cosmétique solide, chauffante, à hydrater, destinée au démaquillage et/ou au nettoyage de la peau, des muqueuses et/ou des yeux. Cette composition peut être appliquée aussi bien sur le visage que sur le corps d'être humain et est d'une très grande douceur. Cette composition de toucher sec a l'aspect de solide déformable et doit être hydratée avant usage.

L'invention se rapporte aussi à un procédé cosmétique de démaquillage et/ou de nettoyage de la peau.

Les compositions démaquillantes pour la peau se présentent habituellement sous forme de lotions, de laits, de gels ou, plus rarement, de crèmes. La consistance de tels produits est fixée par le fabricant et l'utilisateur ne peut faire varier lui-même cette consistance. Or, suivant les utilisateurs et suivant la partie du visage à démaquiller, il est avantageux que l'utilisateur puisse déterminer lui-même la consistance de la composition qu'il va utiliser.

En outre, dans les compositions démaquillantes, il est avantageux de pouvoir incorporer une assez grande quantité d'huiles pour obtenir un démaquillage parfait, les huiles entraînant les matières grasses des produits de maquillage. Or, une trop forte proportion d'huiles rend souvent les compositions démaquillantes de l'art antérieur trop grasses, ce qui entraîne un inconfort rédhibitoire pour l'utilisateur.

Aussi, il subsiste le besoin d'une composition démaquillante dont la consistance puisse être adaptée par chaque utilisateur et/ou qui puisse contenir une quantité suffisante d'huiles.

La présente invention a justement pour objet une nouvelle composition appropriée notamment pour le démaquillage et/ou le nettoyage de la peau, des muqueuses et/ou des yeux, permettant de remédier aux inconvénients mentionnés ci-dessus. Cette composition est une composition chauffante, à hydrater lors de l'utilisation, d'application aisée, qui présente une texture tout à fait inhabituelle, et qui, selon le choix de l'utilisateur, peut être, après hydratation, plus ou moins fluide grâce à l'incorporation d'une quantité d'eau qui varie selon la quantité de composition solide prélevée et selon la consistance souhaitée. Cette composition a, en outre, l'avantage d'être d'un très grand confort malgré la quantité importante d'huile.

Ainsi, l'invention se rapporte à une composition cosmétique solide, chauffante, à hydrater, comprenant une poudre et un liant, la poudre comprenant des particules solides de polymère expansé, et le liant contenant plus de 30 % en poids d'une ou plusieurs huiles, par rapport au poids total de la composition, et une quantité efficace d'un ou plusieurs agents susceptibles de dégager de la chaleur, lors de l'hydratation.

La composition de l'invention est destinée plus spécialement au démaquillage et/ou au nettoyage du visage d'être humain, et plus spécifiquement pour le démaquillage et/ou le nettoyage de la peau, des muqueuses et/ou des yeux. Elle se présente sous forme de solide déformable ou malléable, sec, ne tachant pas et ressemblant à de la guimauve (voir le document US-A-3,682,659 pour la consistance de la guimauve). Ce solide peut être modelé comme de la pâte à modeler pour enfants. Il peut être rompu facilement à la main afin de ne prélever que la quantité nécessaire de produit. En particulier, cette composition peut être conditionnée sous forme de monodose, particulièrement avantageuse d'un point de vue hygiénique, et par exemple sous forme de petits cubes, de billes ou de berlingots.

Grâce à cette texture solide, la composition de l'invention ne risque pas de s'échapper de son conditionnement notamment lors d'un transport. Par ailleurs, son stockage ne pose pas de problème et sa pollution par l'environnement et/ou la manipulation par le consommateur est relativement réduite, et en tout état de cause très inférieure à celle des produits comparables de l'art antérieur. En particulier, il n'est pas absolument nécessaire d'introduire de conservateur pour assurer sa protection antimicrobienne.

On entend ici par « composition chauffante » une composition telle que l'utilisateur ressente un échauffement lors de l'application de la composition hydratée sur la peau. C'est une composition qui, en présence d'eau, peut élever la température du mélange de plusieurs degrés (un à dix degrés) de manière instantanée. Cet effet chauffant va permettre l'ouverture des pores de la peau et donc un meilleur démaquillage.

La composition est quasiment anhydre, c'est-à-dire qu'elle comprend généralement moins de 10 % en poids et de préférence moins de 6 % en poids d'eau, par rapport au poids total de la composition.

La composition de l'invention étant une composition cosmétique et donc destinée à une application topique, elle comporte un milieu physiologiquement acceptable, c'est-à-dire compatible avec la peau, les muqueuses, les yeux et/ou les fibres kératiniques.

Les particules solides de polymère expansé sont de préférence des particules creuses thermoplastiques expansées de polymère ou copolymère formé à partir d'un monomère ou mélange de monomères à insaturation éthylènique.

Les particules utilisables selon l'invention peuvent être réalisées à partir de monomères à insaturation éthylènique, non-toxiques et non-irritants pour la peau.

Les particules de l'invention peuvent être obtenues, par exemple, selon les procédés des brevets et demandes de brevet EP-A-56219, EP-A-348372, EP-A-486080, EP-A-320473, EP-A-112807 et US-A-3615972.

La cavité interne des particules contient en principe un gaz qui peut être de l'air, de l'azote ou un hydrocarbure comme l'isobutane ou l'isopentane.

Parmi les monomères utilisés pour la réalisation des particules creuses thermoplastiques expansées de l'invention, on peut citer les esters d'acide méthacrylique ou acrylique, tels que l'acrylate ou le méthacrylate de méthyle ; le chlorure de vinylidène ; l'acrylonitrile ; le styrène et ses dérivés ; le butadiène et ses dérivés ; leurs mélanges.

On peut par exemple utiliser les polymères ou copolymères d'acrylate ou méthacrylate de méthyle, les copolymères formés à partir de styrène et d'acrylonitrile, les copolymères de chlorure de vinylidène et d'acrylonitrile ou de chlorure de vinyle.

On utilise de préférence un polymère ou copolymère contenant : de 0 à 60 % de chlorure de vinylidène ou d'un de ses dérivés, de 20 à 80 % d'acrylonitrile ou d'un de ses dérivés et de 0 à 50 % d'un monomère (méth)acrylique ou styrènique, la somme des pourcentages (en poids) étant égale à 100. Le monomère (méth)acrylique est par exemple le (méth)acrylate de méthyle ou d'éthyle. Le monomère styrènique est par exemple le styrène ou le α-méthylstyrène.

Plus préférentiellement, les particules utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.

De façon avantageuse, les particules de l'invention ont une granulométrie allant de 1 µm à 300 µm, par exemple de 5 µm à 200 µm et de préférence de 10 µm à 100 µm et mieux de 15 µm à 50 µm.

De façon préférentielle, la masse volumique des particules est choisie dans la gamme allant de 15 kg/m³ à 200 kg/m³ et mieux de 40 kg/m³ à 120 kg/m³, et encore mieux de 60 kg/m³ à 80 kg/m³.

Les particules utilisables dans l'invention sont par exemple les microsphères de terpolymère expansé de chlorure de vinylidène, d'acrylonitrile et de méthacrylate, commercialisées sous la marque EXPANCEL par la société Nobel Casco sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23.

Les particules solides de polymère expansé sont présentes dans les compositions de l'invention dans des concentrations allant, par exemple, de 2 à 30 % en poids, et de préférence de 2 à 10 % en poids par rapport au poids total de la composition.

La composition de l'invention contient dans le liant, une quantité efficace d'un ou plusieurs agents susceptibles de dégager de la chaleur, lors de l'hydratation de la composition. L'agent susceptible de dégager de la chaleur peut être notamment choisi parmi les polyols ayant au moins 2 groupes hydroxyle et au moins 3 atomes de carbone, et parmi les zéolites (activées ou non activées). Ce type d'agent présente la particularité de réagir chimiquement avec l'eau selon un processus exothermique. Pour que ce processus exothermique ait lieu, il est souhaitable que la composition soit quasiment exempte d'eau moins de 6 % en poids par rapport au poids total de la composition).

A titre de polyol ayant au moins 2 groupes hydroxyle et au moins 3 atomes de carbone, on peut citer notamment la glycérine, la diglycérine, le propylène glycol, le butylène glycol, l'hexylène glycol, le polyéthylène glycol et les polyéthylène glycols de poids moléculaire inférieur à 600 comme le PEG-8, les sucres tels que le sorbitol, et leurs mélanges.

A titre de zéolites (silicoaluminates), on peut citer par exemple les zéolites A, les zéolites X comme celles commercialisées par les sociétés Fluka et Union Carbide, les zéolites MAP comme décrit dans le document EP-A-384070, les zéolites A activées telles que décrites dans le document EP-A-187912. Les cations présents dans les zéolites utilisées comprennent notamment Na, K, Ca, Zn, Mg, Li, Cu et leurs combinaisons.

La quantité de l'agent ou des agents susceptibles de dégager de la chaleur doit être telle que l'utilisateur ressente effectivement un échauffement lors de l'application de la composition, après hydratation. En pratique, la quantité d'agent chauffant représente généralement de 10 à 65 % en poids et mieux de 15 à 40 % en poids par rapport au poids total de la composition.

La composition selon l'invention contient plus de 30 % en poids d'une ou plusieurs huiles, par rapport au poids total de la composition. La quantité d'huiles peut aller par exemple de 31 à 50 % en poids et mieux de 31 à 40 % du poids total de la composition. Ces huiles font partie intégrante du liant.

Selon un mode préféré de réalisation de l'invention, la composition de l'invention contient de préférence au moins une huile démaquillante choisie parmi les esters d'acide gras comportant au moins 12 atomes de carbone et les huiles hydrocarbonées à chaîne ramifiée.

Les esters d'acide gras comportant au moins 12 atomes de carbone sont de préférence ceux obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou de di-esters. De manière préférée, ces esters ne comportent aucune insaturation et/ou aucun groupement éther ou hydroxyle. De façon encore plus avantageuse, il s'agit d'un ester saturé qui ne renferme aucun groupement éther ni hydroxyle.

Ainsi, l'ester d'acide gras, utilisé comme huile démaquillante dans la composition conforme à l'invention peut être notamment choisi dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

L'huile hydrocarbonée à chaîne ramifiée utilisable comme huile démaquillante peut être choisie notamment parmi celles comportant de 10 à 20 atomes de carbone et, par exemple, dans le groupe comprenant l'isohexadécane, l'isododécane, les isoparaffines et leurs mélanges.

La composition peut contenir en outre des huiles autres que celles mentionnées ci-dessus. Ces huiles peuvent être choisies parmi les huiles minérales comme l'huile de paraffine et l'huile de vaseline ; les huiles d'origine animale comme le perhydrosqualène ; les huiles d'origine végétale telles que l'huile d'amande douce, d'avocat, de ricin, d'olive, de jojoba, de sésame, d'arachide, de pépins de raisin, de colza, de coprah, de noisettes, de palme, de noyau d'abricot, de calophyllum, de son de riz, de germes de maïs, de germes de blé, de soja, de tournesol, de carthame, de passiflore, de seigle et le beurre de karité et sa fraction liquide ; les huiles de synthèse telles que les triglycérides d'acides gras ; les huiles de silicone telles que les cyclométhicones, les polydiméthylsiloxanes volatiles et/ou non volatiles ou encore les phényldiméthylsiloxanes ; leurs mélanges.

En outre, la composition selon l'invention peut avantageusement contenir un ou plusieurs tensioactifs nettoyants et/ou moussants, qui peuvent être des tensioactifs non ioniques, anioniques, cationiques et/ou amphotères. Ils peuvent être utilisés en une quantité allant par exemple de 0,1 à 15 % du poids total de la composition, et de préférence de 1 à 10 %. De façon avantageuse, le ou les tensioactifs sont sous forme de poudre présentant une granulométrie allant de 5 à 50 µm et mieux de 10 à 20 µm. Ils peuvent aussi se présenter sous forme d'une pâte comportant environ 50 % en poids d'eau.

Comme tensioactifs non ioniques utilisables dans l'invention, on peut citer par exemple les condensats d'oxydes d'alkylène et d'alkyl phénols comme l'octyl phénol éthoxylé tel que celui commercialisé sous le nom Triton X45 par la société Rohm & Haas, les condensats d'oxyde d'éthylène, d'oxyde de propylène et d'éthylène diamine, les alkylpolyglucosides, les éthers d'alcool gras et de polyols comme par exemple le Polyglyceryl-3 hydroxylauryl Ether (nom CTFA) commercialisé sous la dénomination Chimexane NF par la Société Chimex, leurs mélanges.

Comme tensioactifs anioniques, on peut citer par exemple les polyalkylène glycols éther d'alcools gras, les taurates, les acyl lactylates comme le stéaroyl lactylate de sodium (par exemple Pationic SSL commercialisé par la société Maprecos), les alkyl sulfates comme le lauryl sulfate de sodium (Sipon LCS commercialisé par la société Henkel), les alkyl sulfates de glycéryle comme le cocoglycéryl sulfate de sodium (commercialisé par la société Nikko sous le nom Nikkol SGC-80N), les alkyl sulfates polyoxyéthylénés, les alkyl éthers sulfates comme le lauryl éther sulfate de monoéthanolamine, les alkyl éthers carboxylates, les monoalkyl ou dialkyl phosphates comme le monohexyl-2-décyl phosphate d'arginine (MAP-16G-ARG commercialisé par la société Kao Chemicals), les alkyl phosphate éthoxylés, les N-acyl sarcosinates comme le myristoyl sarcosinate de sodium (par exemple Nikkol Sarcosinate MN commercialisé par la société Nikko), les N-acyl glutamates comme le lauroyl glutamate de sodium (par exemple Amisoft LS11 commercialisé par la société Ajinomoto), les acyl iséthionates comme le cocoyl iséthionate de sodium commercialisé notamment par la société Jordan (Jordapon Cl), les polysorbates, les succinamates, les savons comme le laurate, myristate, palmitate ou stéarate de potassium, leurs mélanges.

Comme tensioactifs amphotères ou zwitterions, on peut citer par exemple les bétaïnes et les dérivés de bétaïnes, les sultaïnes et les dérivés de sultaïnes, les dérivés imidazolinium, comme le cocoamphodiacétate de disodium, leurs mélanges.

Comme tensioactifs cationiques utilisables dans l'invention, on peut citer les dérivés de pyrrolidone carboxylate comme le PCA éthyl cocoyl arginate (Cation CAE commercialisé par la société Ajinomoto).

Selon un mode particulier de réalisation de l'invention, le tensioactif est amphotère. Comme tensioactif amphotère, on peut citer plus particulièrement le cocoamphodiacétate de disodium se présentant sous forme d'une pâte aqueuse et commercialisé sous la dénomination Miranol C2M par la société Rhodia Chimie.

La composition peut, en outre, contenir un ou plusieurs autres ingrédients lipophiles, classiquement utilisés dans les compositions de démaquillage. Ces ingrédients sont, notamment, des parfums, des conservateurs, des antioxydants, des agents régulateurs de pH, des séquestrants, des charges, des colorants, des actifs cosmétiques ou dermatologiques, ou leurs mélanges. Ces adjuvants sont utilisés dans les proportions habituelles des compositions nettoyantes et/ou de soin, et par exemple de 0,01 à 10 % du poids total de la composition. Ces adjuvants doivent être de nature et utilisés en quantité telles qu'ils ne perturbent pas les propriétés recherchées pour la composition de l'invention.

Comme charges, on peut citer par exemple le talc, le kaolin, l'amidon modifié ou non.

Selon un mode préféré de réalisation de l'invention, on ajoute comme charge un amidon modifié. Comme amidons modifiés, on peut citer par exemple les amidons estérifiés par l'anhydride octénylsuccinique et plus particulièrement le "Aluminium Starch octenyl succinate" tel que le produit commercialisé par la société NATIONAL STARCH sous le nom de DRY-FLO, l'amidon de maïs réticulé commercialisé sous le nom RESISTAMYL E2 par la société AMYLUM ; l'amidon de pomme de terre estérifié par un groupe carboxyméthyl commercialisé sous le nom SUPRAMYL P 60 par la société AMYLUM, l'amidon de maïs estérifié par un groupe hydroxypropyle commercialisé sous le nom MERIGEL EF6 par la société AMYLUM ; l'amidon prégélatimisé, modifié par l'anhydride octénylsuccinique, puis par un motif hydrophobe, commercialisé sous le nom de NATROSORB HFB par la société NATIONAL STARCH ; l'amidon de maïs réticulé et acétylé commercialisé par la société CERESTAR sous le nom C* Flo 06205 ; l'amidon de pomme de terre réticulé commercialisé par la société Avebe sous le nom PRIMOJEL.

La quantité d'amidon modifié peut aller par exemple de 5 à 40 % et de préférence de 10 à 30 % en poids par rapport au poids total de la composition.

Comme actifs utilisables dans l'invention, on peut citer des antibactériens comme l'octopirox et le triclosan, des agents kératolytiques comme l'acide salicylique, des huiles essentielles, des vitamines notamment lipophiles.

La composition de l'invention est particulièrement adaptée pour le démaquillage et/ou le nettoyage de la peau, des muqueuses et/ou des yeux et plus particulièrement pour le démaquillage ou le nettoyage de la peau.

L'invention a encore pour objet l'utilisation cosmétique de la composition selon l'invention pour le démaquillage et/ou le nettoyage de la peau, des muqueuses et/ou des yeux.

Comme indiqué ci-dessus, la composition de l'invention est sous forme d'un solide déformable et donc malléable, dont on peut prélever la quantité souhaitée. La quantité prélevée est par exemple mise au creux de la main et additionnée d'une quantité d'eau telle que l'on obtienne la consistance souhaitée par l'utilisateur (notamment crème ou lait). On passe la crème ou le lait sur le visage et on rince à l'eau. On obtient un démaquillage particulièrement bon tout en étant très confortable (pas de tiraillements de la peau après démaquillage).

Ainsi, l'invention a encore pour objet un procédé cosmétique de démaquillage et/ou de nettoyage de la peau, consistant à prélever une quantité voulue d'une composition telle que définie ci-dessus, à l'hydrater avec une quantité suffisante d'eau pour avoir la consistance voulue, à appliquer la composition hydratée obtenue sur la peau, et enfin à rincer la peau.

La composition selon l'invention peut être préparée par tout moyen connu de l'homme du métier, et en particulier par simple mélange des différents constituants et moulage dans un moule adéquat. Cependant, de façon avantageuse, elle est préparée par mélange suivi d'un malaxage puis d'une extrusion dans un extrudeur, de préférence un extrudeur bi-vis tel que ceux décrits dans les documents EP-A-605284 et FR-A-2,715,306, et dans lequel les deux vis tournent dans le même sens. On peut aussi utiliser le procédé décrit dans le document EP-A-651991.

L'invention a encore pour objet un procédé de préparation de la composition ci-dessus, consistant à introduire puis malaxer les différents constituants de la composition dans un extrudeur et à mettre en forme le mélange obtenu.

Les différents constituants de la composition sont introduits, à l'entrée de l'extrudeur bi-vis, dans la zone d'alimentation à température ambiante, de préférence environ 20°C. De préférence, on introduit les constituants solides en tête de l'extrudeur, puis les constituants liquides sont introduits latéralement. Le tout est malaxé dans diverses zones de l'extrudeur maintenues à une température allant, préférentiellement, de 15 à 25°C ; la masse obtenue est ensuite transportée vers la sortie de l'extrudeur et extrudée au travers d'une filière. La vitesse de rotation des vis est de l'ordre de 300 à 1000 tours/minute, et va de préférence de 450 à 650 tours/minute.

La masse extrudée sort de la filière sous la forme de boudins de diamètre donné selon la filière utilisée, pouvant être ensuite découpés et mis en forme, notamment, sous forme de bâton ou de pain solide. D'autres formes peuvent bien entendu être réalisées en choisissant des filières appropriées et des dispositifs de mise en forme des produits finaux, adaptés à la forme recherchée.

La masse extrudée peut également être déshydratée et/ou broyée et/ou compactée. La déshydratation est avantageusement utilisée lorsque les constituants de la composition sont introduits sous forme de solution ou de dispersion en milieu aqueux.

Du fait que la totalité du procédé d'extrusion est réalisée à température ambiante, de l'ordre de 20-25°C, il est possible d'utiliser des ingrédients sensibles à la chaleur, du type vitamines ou huiles volatiles.

D'autre part, les ingrédients sensibles à la chaleur peuvent être introduits dans n'importe quelle zone de l'extrudeur (en tête, au milieu ou en final) puisqu'aucune détérioration due à la chaleur n'est à craindre. Il est également possible d'effectuer une partie de l'extrusion sous gaz inerte (azote par exemple).

L'exemple ci-après est donné à titre illustratif et non limitatif en vue de mieux faire ressortir les caractéristiques de l'invention. Les quantités y sont données en % en poids.

### Exemple : Composition démaquillante chauffante

- Disodium Cocoamphodiacétate (à 50 % de matière active)
   (Miranol C2M commercialisé par la société Rhodia Chimie) 8 %
   (soit 4 % de matière active)
- PEG-8 9,5 %
- Propylène glycol 9,5 %
- Amidon réticulé (PRIMOJEL commercialisé par la société Avebe) 20 %
- Palmitate d'éthyl-2-hexyle 15 %
- Isododécane 20 %
- Kaolin 13 %
- Expancel 551 DE 20 5 %

Mode opératoire : on mélange le tensioactif (Disodium Cocoamphodiacétate) avec les huiles (palmitate d'éthyl-2-hexyle et isododécane) (phase A). Par ailleurs, on mélange les glycols (phase B). On ajoute l'amidon réticulé à la phase A, sous agitation, puis on y ajoute le phase B et enfin le kaolin. Le mélange obtenu et l'Expancel sont introduits dans un extrudeur dont la vitesse de rotation est de 450 tours/minute. Le produit est mis en forme dans l'extrudeur grâce à une filière.

Le produit obtenu est une pâte non collante, facilement modelable, facilement mouillable ou hydratable, d'application et d'élimination faciles, douce au toucher et ayant un bon pouvoir démaquillant.

Ce produit peut alors être hydraté, puis malaxé notamment dans le creux de la main. La quantité d'eau dépend de la consistance que l'on souhaite obtenir (composition plus ou moins fluide) et elle peut représenter de 6 à 10 fois en volume celle de l'échantillon prélevé pour le nettoyage.

Dès l'application de la composition sur le visage, on ressent un léger dégagement de chaleur, assurant l'ouverture des pores de la peau et la libération des impuretés, tandis que les huiles assurent l'élimination des produits gras de maquillage. L'élimination de la composition se fait aisément avec de l'eau.

## Revendications

1. Composition cosmétique solide, chauffante, à hydrater, comprenant une poudre et un liant, la poudre comprenant des particules solides de polymère expansé, et le liant contenant plus de 30 % en poids d'une ou plusieurs huiles, par rapport au poids total de la composition, et une quantité efficace d'un ou plusieurs agents susceptibles de dégager de la chaleur, lors de l'hydratation.

2. Composition selon la revendication 1, **caractérisée en ce que** les particules sont des particules creuses thermoplastiques expansées de polymère ou copolymère formé à partir d'un monomère ou d'un mélange de monomères à insaturation éthylènique.

3. Composition selon la revendication 1 ou 2, **caractérisée en ce que** les particules sont des particules de polymère ou de copolymère expansé formé à partir d'un monomère ou d'un mélange de monomères choisis dans le groupe formé par les esters d'acide méthacrylique ou acrylique ; le chlorure de vinylidène ; l'acrylonitrile ; le styrène et ses dérivés ; le butadiène et ses dérivés ; et leurs mélanges.

4. Composition selon l'une quelconque des revendications précédentes, **caractérisée en ce que** les particules sont des particules creuses d'un copolymère expansé de chlorure de vinylidène et d'acrylonitrile ou un terpolymère expansé de chlorure de vinylidène, d'acrylonitrile et de méthacrylate de méthyle.

5. Composition selon l'une quelconque des revendications précédentes, **caractérisée en ce que** les particules ont une granulométrie de 1 à 300 µm.

6. Composition selon l'une quelconque des revendications précédentes, **caractérisée en ce que** les particules ont une masse volumique allant de 15 à 200 kg/m³.

7. Composition selon l'une quelconque des revendications précédentes, **caractérisée en ce qu'**elle contient de 2 à 30 % en poids de particules solides de polymère expansé, par rapport au poids total de la composition.

8. Composition selon l'une quelconque des revendications précédentes, **caractérisée en ce que** l'agent susceptible de dégager de la chaleur est choisi parmi les polyols, les zéolites ou leurs mélanges.

9. Composition selon l'une des revendications précédentes, **caractérisée en ce que** l'agent susceptible de dégager de la chaleur est choisi parmi la glycérine, la diglycérine, les polyéthylène glycols, le propylène glycol, le butylène glycol, l'hexylène glycol, les sucres et leurs mélanges.

10. Composition selon l'une des revendications précédentes, **caractérisée en ce que** le ou les agents susceptibles de dégager de la chaleur sont présents en une quantité allant de 10 à 65 % du poids total de la composition.

11. Composition selon l'une des revendications précédentes, **caractérisée en ce qu'**elle contient une quantité d'une ou plusieurs huiles, allant de 31 à 50 % en poids par rapport au poids total de la composition.

12. Composition selon l'une quelconque des revendications précédentes, **caractérisée en ce qu'**elle contient au moins une huile démaquillante choisie parmi les esters d'acide gras comportant au moins 12 atomes de carbone et les huiles hydrocarbonées à chaîne ramifiée.

13. Composition selon l'une des revendications précédentes, **caractérisée en ce qu'**elle contient, en outre, au moins un tensioactif moussant et/ou nettoyant.

14. Composition selon la revendication précédente, **caractérisée en ce que** le tensioactif est un tensioactif amphotère.

15. Composition selon la revendication 13 ou 14, **caractérisée en ce que** la quantité de tensioactif va de 0,1 à 15 % en poids par rapport au poids total de la composition.

16. Composition selon l'une quelconque des revendications précédentes, **caractérisée en ce qu'**elle contient de l'amidon modifié.

17. Composition selon la revendication précédente, **caractérisée en ce que** la quantité d'amidon modifié va de 5 à 40 % en poids par rapport au poids total de la composition.

18. Composition selon l'une des revendications précédentes, **caractérisée en ce qu'**elle se présente sous forme de solide déformable.

19. Composition selon l'une des revendications précédentes, **caractérisée en ce qu'**elle est obtenue par extrusion à froid.

20. Utilisation cosmétique de la composition selon l'une des revendications précédentes pour le démaquillage et/ou le nettoyage de la peau, des muqueuses et/ou des yeux.

21. Procédé cosmétique de démaquillage et/ou de nettoyage de la peau, consistant à prélever une quantité voulue d'une composition selon l'une quelconque des revendications 1 à 19, à l'hydrater avec une quantité suffisante d'eau pour avoir la consistance voulue, à appliquer la composition hydratée obtenue sur la peau, et enfin à rincer la peau.

22. Procédé de préparation de la composition selon l'une des revendications 1 à 19, consistant à introduire puis malaxer les différents constituants de la composition dans un extrudeur et à mettre en forme le mélange obtenu.

## Patentansprüche

1. Feste, Wärme entwickelnde, kosmetische Zusammensetzung zum Anfeuchten, die ein Pulver und ein Bindemittel enthält, wobei das Pulver feste Partikel eines expandierten Polymers und das Bindemittel mehr als 30 Gew.-% eines oder mehrerer Öle, bezogen auf das Gesamtgewicht der Zusammensetzung, und eine wirksame Menge eines oder mehrerer Stoffe enthält, die befähigt sind, bei der Hydratisierung Wärme zu entwickeln.

2. Zusammensetzung nach Anspruch 1, **dadurch gekennzeichnet, dass** die Partikel thermoplastische expandierte Hohlpartikel eines Polymers oder Copolymers sind, das ausgehend von einem Monomer oder einem Gemisch von Monomeren mit ethylenisch ungesättigter Bindung gebildet wird.

3. Zusammensetzung nach Anspruch 1 oder 2, **dadurch gekennzeichnet, dass** die Partikel Partikel eines expandierten Polymers oder Copolymers sind, das aus einem Monomer oder einem Gemisch von Monomeren gebildet wird, die unter Methacrylsäureestern oder Acrylsäureestern; Vinylidenchlorid; Acrylnitril; Styrol und seinen Derivaten; Butadien und seinen Derivaten; und deren Gemischen ausgewählt sind.

4. Zusammensetzung nach einem der vorhergehenden Ansprüche, **dadurch gekennzeichnet, dass** die Partikel Hohlpartikel eines expandierten Copolymers von Vinylidenchlorid und Acrylnitril oder eines expandierten Terpolymers von Vinylidenchlorid, Acrylnitril und Methylmethacrylat sind.

5. Zusammensetzung nach einem der vorhergehenden Ansprüche, **dadurch gekennzeichnet, dass** die Partikel eine Teilchengröße von 1 bis 300 µm besitzen.

6. Zusammensetzung nach einem der vorhergehenden Ansprüche, **dadurch gekennzeichnet, dass** die Partikel eine Dichte von 15 bis 200 kg/m³ aufweisen.

7. Zusammensetzung nach einem der vorhergehenden Ansprüche, **dadurch gekennzeichnet, dass** sie 2 bis 30 Gew.-% feste Partikel eines expandierten Copolymers, bezogen auf das Gesamtgewicht der Zusammensetzung, enthält.

8. Zusammensetzung nach einem der vorhergehenden Ansprüche, **dadurch gekennzeichnet, dass** der Stoff, der befähigt ist, Wärme zu entwickeln, unter den Polyolen, Zeolithen und deren Gemischen ausgewählt ist.

9. Zusammensetzung nach einem der vorhergehenden Ansprüche, **dadurch gekennzeichnet, dass** der Stoff, der Wärme entwickeln kann, unter Glycerin, Diglycerin, Polyethylenglykolen, Propylenglykol, Butylenglykol, Hexylenglykol, Zuckern und deren Gemischen ausgewählt ist.

10. Zusammensetzung nach einem der vorhergehenden Ansprüche, **dadurch gekennzeichnet, dass** der Stoff oder die Stoffe, die befähigt sind, Wärme zu entwickeln, in einer Menge von 10 bis 65 % des Gesamtgewichts der Zusammensetzung vorliegen.

11. Zusammensetzung nach einem der vorhergehenden Ansprüche, **dadurch gekennzeichnet, dass** sie ein oder mehrere Öle in einer Menge von 31 bis 50 Gew.-%, bezogen auf das Gesamtgewicht der Zusammensetzung, enthält.

12. Zusammensetzung nach einem der vorhergehenden Ansprüche, **dadurch gekennzeichnet, dass** sie mindestens ein Öl zum Abschminken enthält, das unter den Fettsäureestern mit mindestens 12 Kohlenstoffatomen und den Kohlenwasserstoffölen mit verzweigter Kette ausgewählt ist.

13. Zusammensetzung nach einem der vorhergehenden Ansprüche, **dadurch gekennzeichnet, dass** sie ferner mindestens einen schäumenden und/oder reinigenden grenzflächenaktiven Stoff enthält.

14. Zusammensetzung nach dem vorhergehenden Anspruch, **dadurch gekennzeichnet, dass** der grenzflächenaktive Stoff ein amphoterer grenzflächenaktiver Stoff ist.

15. Zusammensetzung nach Anspruch 13 oder 14, **dadurch gekennzeichnet, dass** der Mengenanteil des grenzflächenaktiven Stoffes im Bereich von 0,1 bis 15 Gew.-%, bezogen auf das Gesamtgewicht der Zusammensetzung, liegt.

16. Zusammensetzung nach einem der vorhergehenden Ansprüche, **dadurch gekennzeichnet, dass** sie eine modifizierte Stärke enthält.

17. Zusammensetzung nach dem vorhergehenden Anspruch, **dadurch gekennzeichnet, dass** der Mengenanteil der modifizierten Stärke im Bereich von 5 bis 40 Gew.-%, bezogen auf das Gesamtgewicht der Zusammensetzung, liegt.

18. Zusammensetzung nach einem der vorhergehenden Ansprüche, **dadurch gekennzeichnet, dass** sie als verformbarer Feststoff vorliegt.

19. Zusammensetzung nach einem der vorhergehenden Ansprüche, **dadurch gekennzeichnet**, daas sie durch Extrusion auf kaltem Wege hergestellt wird.

20. Kosmetische Verwendung der Zusammensetzung nach einem der vorhergehenden Ansprüche zum Abschminken und/oder zur Reinigung der Haut, der Schleimhäute und/ oder der Augen.

21. Kosmetisches Verfahren zum Abschminken und/oder zur Reinigung der Haut, das darin besteht, die gewünschte Menge einer Zusammensetzung nach einem der Ansprüche 1 bis 19 zu entnehmen, mit einer für die gewünschte Konsistenz ausreichenden Wassermenge anzufeuchten, die erhaltene hydratisierte Zusammensetzung auf die Haut aufzutragen und schließlich mit Wasser zu spülen.

22. Verfahren zur Herstellung der Zusammensetzung nach einem der Ansprüche 1 bis 19, das darin besteht, die verschiedenen Bestandteile der Zusammensetzung in einen Extruder einzubringen und dann zu kneten und das erhaltene Gemisch zu formen.

## Claims

1. Solid heating cosmetic composition to be hydrated, comprising a powder and a binder, the powder comprising solid particles of expanded polymer and the binder containing more than 30% by weight of one or more oils, relative to the total weight of the composition, and an effective amount of one or more agents capable of releasing heat, during the hydration.

2. Composition according to Claim 1, **characterized in that** the particles are expanded thermoplastic hollow particles of polymer or copolymer formed from an ethylenically unsaturated monomer or mixture of monomers.

3. Composition according to Claim 1 or 2, **characterized in that** the particles are particles of expanded polymer or copolymer formed from a monomer or a mixture of monomers chosen from the group formed by methacrylic or acrylic acid esters; vinylidene chloride; acrylonitrile; styrene and its derivatives; butadiene and its derivatives; and mixtures thereof.

4. Composition according to any one of the preceding claims, **characterized in that** the particles are hollow particles of an expanded copolymer of vinylidene chloride and of acrylonitrile or an expanded terpolymer of vinylidene chloride, of acrylonitrile and of methyl methacrylate.

5. Composition according to any one of the preceding claims, **characterized in that** the particles have a particle size of from 1 to 300 µm.

6. Composition according to any one of the preceding claims, **characterized in that** the particles have a density ranging from 15 to 200 kg/m³.

7. Composition according to any one of the preceding claims, **characterized in that** it contains from 2% to 30% by weight of solid particles of expanded polymer, relative to the total weight of the composition.

8. Composition according to any one of the preceding claims, **characterized in that** the agent capable of releasing heat is chosen from polyols and zeolites, or mixtures thereof.

9. Composition according to one of the preceding claims, **characterized in that** the agent capable of releasing heat is chosen from glycerol, diglycerol, polyethylene glycols, propylene glycol, butylene glycol, hexylene glycol and sugars, and mixtures thereof.

10. Composition according to one of the preceding claims, **characterized in that** the agent(s) capable of releasing heat is (are) present in an amount ranging from 10% to 65% relative to the total weight of the composition.

11. Composition according to one of the preceding claims, **characterized in that** it contains an amount of one or more oils ranging from 31% to 50% by weight relative to the total weight of the composition.

12. Composition according to any one of the preceding claims, **characterized in that** it contains at least one makeup-removing oil chosen from fatty acid esters containing at least 12 carbon atoms, and branched-chain hydrocarbon-based oils.

13. Composition according to one of the preceding claims, **characterized in that** it also contains at least one foaming and/or cleansing surfactant.

14. Composition according to the preceding claim, **characterized in that** the surfactant is an amphoteric surfactant.

15. Composition according to Claim 13 or 14, **characterized in that** the amount of surfactant ranges from 0.1% to 15% by weight relative to the total weight of the composition.

16. Composition according to any one of the preceding claims, **characterized in that** it contains modified starch.

17. Composition according to the preceding claim, **characterized in that** the amount of modified starch ranges from 5% to 40% by weight relative to the total weight of the composition.

18. Composition according to one of the preceding claims, **characterized in that** it is in the form of a deformable solid.

19. Composition according to one of the preceding claims, **characterized in that** it is obtained by cold extrusion.

20. Cosmetic use of the composition according to one of the preceding claims, for removing makeup from and/or cleansing the skin, mucous membranes and/or the eyes.

21. Cosmetic process for removing makeup from and/or cleansing the skin, which consists in taking a desired amount of a composition according to any one of Claims 1 to 19, hydrating it with a sufficient amount of water to have the desired consistency, applying the hydrated composition obtained onto the skin, and finally rinsing the skin.

22. Process for preparing the composition according to any one of Claims 1 to 19, which consists in introducing into and then blending the various constituents of the composition in an extruder and shaping the mixture obtained.
